# EUROPEAN PATENT SPECIFICATION

(11) **EP 2 329 781 B1**
(45) Date of publication and mention of the grant of the patent: **04.03.2015**
(21) Application number: 09177910.8
(22) Date of filing: 03.12.2009
(51) Int. Cl.: A61B 17/86

(54) **Bone screw**
Knochenschraube
Vis à os

(43) Date of publication of application: 08.06.2011
(60) Divisional application: 13162625.1
(73) Proprietor: Biedermann Technologies GmbH & Co. KG, 78166 Donaueschingen (DE)
(72) Inventor: Biedermann, Lutz, 78048, VS-Villingen (DE); Matthis, Wilfried, 79367, Weisweil (DE); Harms, Jürgen, 76227, Karlsruhe (DE)
(74) Representative: Prüfer & Partner GbR European Patent Attorneys

(56) References cited:
- WO-A2-2004/069031
- JP-A- 10 211 213
- US-A1- 2001 007 072
- US-A1- 2005 107 791
- US-A1- 2007 162 028
- US-A1- 2009 210 016

## Description

The invention relates to a bone screw. In particular, the invention relates to a bone screw which can be used as a fusion screw. This screw promotes fusion in the surrounding bone.

A bone screw of this kind is known from US 2004/0015172 A1. This bone screw has a tubular thread section with a bone thread and with a plurality of recesses in its wall. A head and a tip can be connected to the tubular thread section. In use, the tubular portion can be filled with bone material or other growth-promoting material and then the tip and/or the head are connected to the tubular portion. First, a core hole is prepared, then the screw is inserted into the core hole and screwed into the bone. After a certain period, fusion between the screw and the bone takes place. The screw can act as a traction element to connect shattered or split off parts of bones together by means of the screw. To avoid the preparation of a core hole, the tip can be self-cutting.

US 2004/0122431 describes a bone screw of the kind mentioned above, however without a head. Such a bone screw can be used for example together with a marrow nail. US 2005/0055026 A1 describes a bone screw of the type mentioned above wherein a head which is a separate part can be connected to a tubular threaded shank. The head is configured to be connected to a stabilization rod. In one embodiment, the tubular threaded shank of the screw has at its end which is to be connected to the head slits for engagement with a screw driver.

Further, so-called injection screws, for example such as described in WO 01/26568 A1 are known which are used to inject bone cement or other liquid or pasty material into the bone. This kind of screw has a relatively thin cannula entending within a bone screw. The cannula is not suitable for being filled with bone chips or bone graft.

US 2001/0007072 A1 discloses a bone anchoring assembly comprising a longitudinal circular cylindrical anchoring part, a connecting element, an external thread, wherein.the anchoring part is self cutting at the lower end.

It is the object of the invention to provide a bone screw which can be quickly screwed into the bone without using a prepared core hole.

The object is solved by a bone screw according to claim 1. Further developments are given in the dependent claims.

The bone screw according to the invention is self-filling. When it is screwed into the bone the cutting teeth engage the bone and create a hole. The bone material from the location at which the screw is inserted fills the cavity of the tubular body. Hence, the preparation of a core hole is not necessary. It is not necessary to pre-fill the tubular body with bone material. Therefore, the procedure of implanting the bone screw is less time consuming than in a case where the bone screw has to be pre-filled and inserted into a core hole prepared in advance. Furthermore, the bone material has a more intact structure compared to pre-filled bone material which could enhance the healing process.

The bone screw is particularly suitable for strong healthy bones and for weak osteoporotic bones, since the structural damage which occurs when screwing-in the bone screw can be kept to a minimum.

By using a guide wire procedure for insertion the bone screw can be used in minimally invasive surgery.

Further features and advantages of the invention will become apparent from the description of various embodiments of the invention by means of the accompanying drawings.

In the drawings:
- Fig. 1: shows a perspective view of a bone screw according to a first embodiment.
- Fig. 2: shows a top view of the bone screw according to Fig. 1.
- Fig. 3: shows a sectional view of a bone screw according to Fig. 1, the section being taken in a plane containing the longitudinal axis L of the bone screw.
- Fig. 4: shows a side view of the bone screw of Fig. 1.
- Fig. 5: shows a perspective view of a bone screw according to a second embodiment.
- Fig. 6: shows a top view of the bone screw of Fig. 5.
- Fig. 7: shows a sectional view of the bone screw of Fig. 5, the section being taken in a plane containing the longitudinal axis L of the bone screw.
- Fig. 8: shows a side view of the bone screw of Fig. 5.
- Fig. 9: shows a perspective view of the bone screw of Figs. 5 to 8 together with a guide wire and a tool for insertion.
- Fig. 10: shows a sectional view of the tool which comprises an engagement structure.

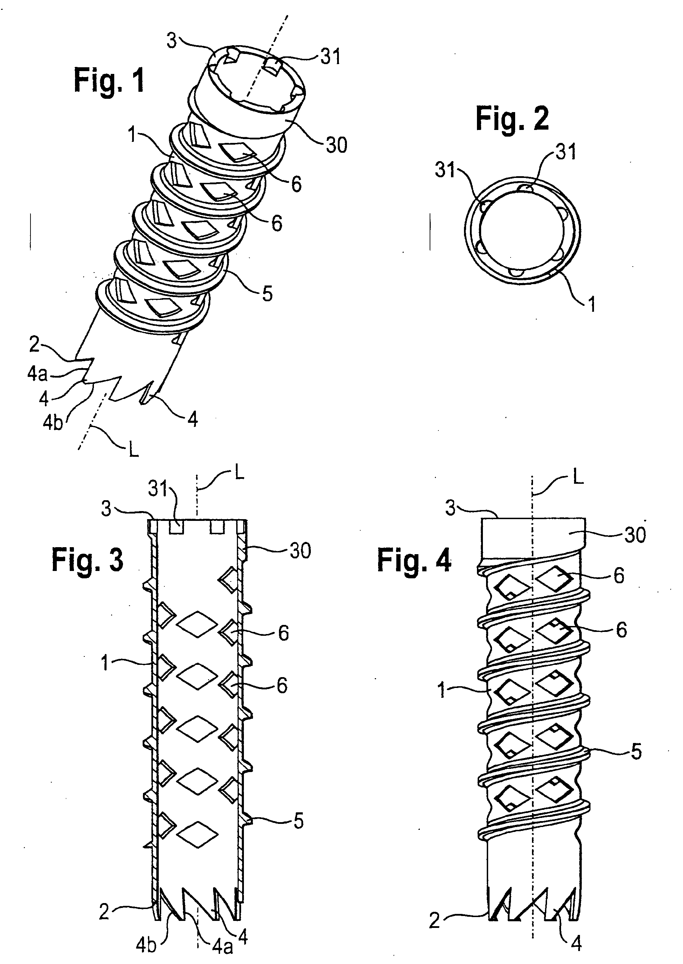

A bone screw according to a first embodiment as shown in Figs. 1 to 4 comprises a tubular body 1 with an open first end 2 and an open second end 3 and a central longitudinal axis L. At the first end 2 the free edge of the tubular body comprises a plurality of cutting teeth 4 which are configured to cut a hole into the bone. In a portion of its outer wall, as shown in the embodiment this portion is close to the first end 2, the tubular body comprises a so-called bone thread 5. The bone thread 5 is configured to cut into the bone when the bone screw is screwed-in into the bone. Although, the Figures show a bone thread with a substantially saw-tooth shape, all kind of known bone threads can be used. The cutting teeth 4 extend from the edge of first end 2 coaxially to the longitudinal axis L. In the embodiment shown, they are substantially saw-tooth shaped. This shape includes a steep flank 4a which extends under an angle of larger than 45° with respect to a circumferential line along the free edge and a more shallow flank 4b which extends under an angle of greater than 0° up to 45° with respect to the free edge. In order ease the insertion of the screw, the steep flanks are oriented all in the same circumferential direction. The cutting teeth are configured to cut a ring-shaped hole into the bone. Within the hole, bone material remains. The bone material which remains in-between the cutting teeth is accommodated in the tubular body. It can consist either of chips or it can be a plug-like coherent mass or both.

The thickness of the cutting teeth in a radial direction is larger than a thickness of the wall of the tubular body (not shown). This results in providing an enlarged space within the tubular body to accommodate the bone material.

The height, the shape and the number of the cutting teeth can vary. All kinds of cutting teeth are suitable which are configured to cut the bone so that bone material remains inside the tubular body.

In a wall of the tubular body, a plurality of openings 6 are provided to allow in-growth of bone material and vessels from the surrounding of the bone screw. The opening are shown as diamond-shaped and extend completely through the wall of the tubular body 1. They are located between the crests 5a of the bone thread 5. Any other variations of the shapes and locations of the openings 6 are conceivable.

Adjacent the second open end 3 a section 30 of the tubular body 1 is formed which is non-threaded. The section 30 comprises at its inner wall a plurality of recesses 31 equally spaced in a circumferential direction which form an engagement structure for a screw driver or another tool. The recesses 31 extend from the edge of the open end 3 to a certain distance therefrom. The wall thickness of the tubular body 1 in the section 30 having the engagement structure may be enhanced compared to the wall thickness of the remaining tubular body 1.The recesses shown in the Figures form a star-like engagement structure for a star screw driver. However, any other shape of the engagement structure such as a hexagon socket or square socket or any other polygon socket is conceivable.

Although the tubular body is shown to be cylindrically-shaped, other shapes are conceivable. For example, the tubular body 1 may have close to the first end 2 a reversed tapered section tapering away from the first end 2. The cavity provided by the tubular body has a volume which is suitable for accommodating bone material. The wall thickness of the tubular body is preferably smaller than about 15% of the screw core diameter.

All parts of the bone screw are made of a body compatible material such as a body compatible metal, for example stainless steel or titanium, a body compatible metal alloy, for example Nitinol or a body compatible plastic material, for example PEEK.

In addition, the tubular body or the other parts of the bone screw can be coated with an in-growth promoting material or can be roughened to enhance in-growth of bone or vessels.

In use, the bone screw according to the first embodiment is inserted into the bone by engaging the engagement structure 31 with a screw driver. The cutting teeth 4 generate the hole for the screw and the bone thread 5 facilitates further advancement of the bone screw into the hole. Bone material which is scraped-off by the cutting teeth 4 fills the interior of the tubular body 1. After a certain time period, the bone material in the tubular body fuses with bone material surrounding the bone screw so that the bone screw is rigidly connected to the bone.

Since the bone screw does not have a head it is not used as a tension screw which is fused for reposition of bone fragments. The bone screw according to this embodiment is used for the immobilization of joints, for example for the immobilization of facet joints.

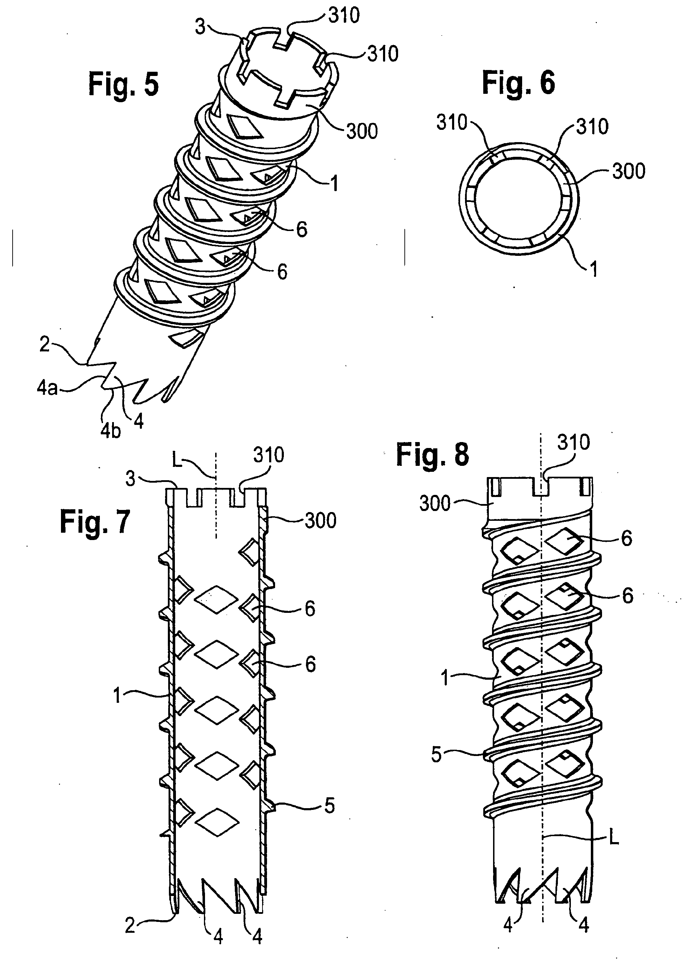

In Figs. 5 to 8 a second embodiment of the bone screw is shown. The second embodiment differs from the first embodiment in the design of the engagement structure for the tool. All other portions are similar to the first embodiment and are designated with the same reference numerals. The description thereof is not repeated.

The bone screw according to the second embodiment has adjacent its open second end 3 a non-threaded portion 300 which comprises a plurality of slits 310 extending from the edge of the open end to a certain distance in longitudinal direction of the tubular body 1. The slits 310 extend completely through the wall so that they form an engagement structure which can be engaged with a corresponding engagement structure of a tool, for example with pins of a tool. The shape of the slits 310 is shown as rectangular. However, any other shape is conceivable which allows to transmit torque when the engagement structure of the tool engages the slits. The number and dimension of the slits can vary.

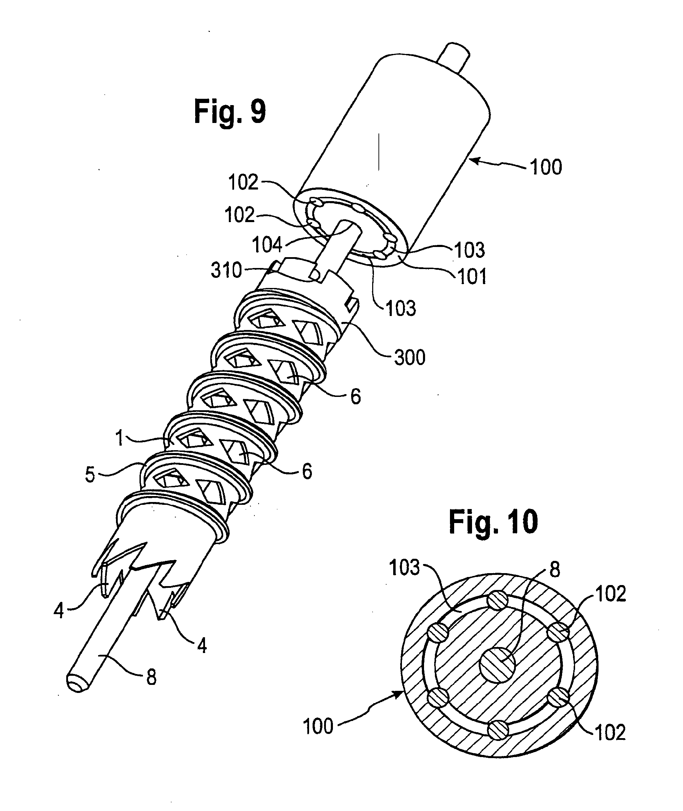

Figs. 9 and 10 show the bone screw according to the second embodiment together with a tool 100 and a guide wire 8. The tool 100 is for example a cylinder which is adapted to cooperate with the engagement structure of the bone screw. At the front face 101 a plurality of pins 102 are provided which are configured so as to engage the recesses 310 of the engagement structure of the bone screw. Further, a groove 103 is provided in which the free edge of the open end 3 of the tubular body of the bone screw can be inserted to facilitate the appliance of the tool 100. The pins 102 are located at equal distances in the groove and do not or do not much project out of the front face 101. The tool 100 further comprises a coaxial guide hole 104 for guiding the guide wire 8 therethrough. The length of the tool 100 is such that the guide wire 8 is guided by the tool 100 in a stabile manner.

Although the tool is shown to be cylindrical, it can have any other shape and any other engagement structure which cooperates with the engagement structure of the bone screw. For example, the engagement structure can be designed so as to engage the engagement structure of the bone screw according to the first embodiment.

The bone screw with the tool and the guide wire as shown in Figs. 9 and 10 can be used in minimally invasive surgery (MIS). First, the guide wire which is guided by the tool 100 is inserted into the tubular body 1 as shown in Fig. 9. Then, the guide wire 8 is introduced through the skin of the patient and advanced through the tissue until it reaches the position where the bone screw has to be placed. Then, the guide wire is inserted into the bone to the appropriate direction and depth. Then, the bone screw is guided along the guide wire 8 extending therethrough until it reaches the surface of the bone. Next, the bone screw is screwed into the bone guided by the guide wire by rotating the tool 100. The cutting teeth 4 generate the hole for the screw and the bone thread 5 facilitates further advancement of the bone screw into the hole. Bone material which is scraped-off by the cutting teeth 4 fills the interior of the tubular body 1. When the bone screw is finally implanted, the guide wire is retracted. After a certain time period, the bone material in the tubular body fuses with bone material surrounding the bone screw so that the bone screw is rigidly connected to the bone. The bone screw can be used as the bone screw according to the first embodiment particularly for stabilizing joints.

## Claims

1. Bone screw including
a tubular body (1) with a bone thread (5) in at least a portion of its outer wall, the tubular body having an open first end (2) and an open second end (3);
wherein at the open first end (2) a plurality of cutting teeth (4) is provided and
wherein at the second end (3) a structure (31, 310) for engagement with a screwing-in tool (100) is provided; **characterized in that** the cutting teeth (4) are thicker in a radial direction compared to the wall of the tubular body.

2. Bone screw according to claim 1, wherein the cutting teeth (4) extend coaxially to the central axis (L) of the tubular body.

3. Bone screw according to claim 1 or 2, wherein the cutting teeth (4) are configured to cut a hole when the tubular body (1) is screwed into the bone.

4. Bone screw according to one of claims 1 to 3, wherein the cutting teeth (4) have a substantially saw tooth shape.

5. Bone screw according to one of claims 1 to 4, wherein the cutting teeth (4) form an edge of the open first end (2).

6. Bone screw according to one of claims 1 to 5, wherein the wall thickness of the tubular body is smaller than about 15% of the outer diameter of the tubular body.

7. Bone screw according to one of claims 1 to 6, wherein the engagement structure comprises a plurality of recesses (31) extending from the open second end (3) through the wall of the tubular body substantially coaxially to a central longitudinal axis (L) of the tubular body.

8. Bone screw according to one of claims 1 to 7, wherein the engagement structure comrises a polygon recess or is a star-like recess (31) extending from the open second end (3) into the inner wall of the tubular body.

9. Bone screw according to one of claims 1 to 6, wherein adjacent to the second open end (3) a section (300) of the tubular body (1) is formed which is non-threaded, wherein the section (300) comprises a plurality of slits (310) extending from the edge of the open end (3) to a certain distance in longitudinal direction of the tubular body (1), wherein the slits (310) extend completely through the wall, and wherein the slits (310) form an engagement structure for a tool.

10. Bone screw according to one of claims 1 to 9, wherein the wall of the tubular body comprises a plurality of openings (6).

11. Bone screw according to one of claims 1 to 8 or 10, wherein the tubular body comprises a non threaded section.

12. Bone screw according to one of claims 1 to 11, wherein a guide wire (8) is provided which is insertable into the tubular body for guiding the insertion of the bone screw.

13. Bone screw according to one of claims 1 to 12, wherein the tubular body (1) is cylindrical.

14. Bone screw according to one of claims 1 to 13 together with a tool for inserting said bone screw, wherein the tool comprises an engagement structure (102, 103) which engages the engagement structure (31, 310) of the bone screw and which is configured to guide a guide wire.

15. The combination according to claim 14, wherein the tool comprises a guide hole (104) for guiding the guide wire therethrough.

## Patentansprüche

1. Knochenschraube umfassend
einen rohrförmigen Körper (1) mit einem Knochengewinde (5) auf wenigstens einem Abschnitt seiner äußeren Wand, wobei der rohrförmige Körper ein offenes erstes Ende (2) und ein offenes zweites Ende (3) aufweist;
wobei an dem offenen ersten Ende (2) eine Vielzahl von Schneidezähnen (4) vorgesehen ist und
wobei an dem zweiten Ende (3) eine Struktur (31, 310) zum Eingreifen mit einem Einschraubwerkzeug (100) vorgesehen ist; **dadurch gekennzeichnet, dass** die Schneidezähne (4) in einer radialen Richtung dicker sind im Vergleich zu der Wand des rohrförmigen Körpers.

2. Knochenschraube gemäß Anspruch 1, wobei sich die Schneidezähne (4) koaxial zu der Zentralachse (L) des rohrförmigen Körpers erstrecken.

3. Knochenschraube gemäß Anspruch 1 oder 2, wobei die Schneidezähne (4) zum Schneiden eines Loches ausgebildet sind, wenn der rohrförmige Körper (1) in den Knochen geschraubt wird.

4. Knochenschraube gemäß einem der Ansprüche 1 bis 3, wobei die Schneidezähne (4) eine im Wesentlichen Sägezahnform aufweisen.

5. Knochenschraube gemäß einem der Ansprüche 1 bis 4, wobei die Schneidezähne (4) eine Kante des offenen ersten Endes (2) bilden.

6. Knochenschraube gemäß einem der Ansprüche 1 bis 5, wobei die Wanddicke des rohrförmigen Körpers kleiner ist als etwa 15% des Außendurchmessers des rohrförmigen Körpers.

7. Knochenschraube gemäß einem der Ansprüche 1 bis 6, wobei die Eingreifstruktur eine Vielzahl von Ausnehmungen (31) umfasst, welche sich im Wesentlichen koaxial von dem offenen zweiten Ende (3) durch die Wand des rohrförmigen Körpers zu der zentralen Längsachse (L) des rohrförmigen Körpers erstrecken.

8. Knochenschraube gemäß einem der Ansprüche 1 bis 7, wobei die Eingreifstruktur eine polygone oder eine sternförmige Ausnehmung (31) umfasst, welche sich von dem offenen zweiten Ende (3) in die innere Wand des rohrförmigen Körpers erstreckt.

9. Knochenschraube gemäß einem der Ansprüche 1 bis 6, wobei angrenzend an das zweite offene Ende (3) ein Abschnitt (300) des rohrförmigen Körpers (1) ausgebildet ist, welcher gewindefrei ist, wobei der Abschnitt (300) eine Mehrzahl von Schlitzen (310) umfasst, welche sich vom Rand des offenen Endes (3) zu einer bestimmten Distanz in Längsrichtung des rohrförmigen Körpers (1) erstrecken, wobei sich die Schlitze (310) vollständig durch die Wand erstrecken und wobei die Schlitze (310) eine Eingreifstruktur für ein Werkzeug bilden.

10. Knochenschraube gemäß einem der Ansprüche 1 bis 9, wobei die Wand des rohrförmigen Körpers eine Mehrzahl von Öffnungen (6) umfasst.

11. Knochenschraube gemäß einem der Ansprüche 1 bis 8 oder 10, wobei der rohrförmige Körper einen gewindefreien Abschnitt umfasst.

12. Knochenschraube gemäß einem der Ansprüche 1 bis 11, wobei ein Führungsdraht (8) vorgesehen ist, welcher in den rohrförmigen Körper zum Führen des Einbringens der Knochenschraube einführbar ist.

13. Knochenschraube gemäß einem der Ansprüche 1 bis 12, wobei der rohrförmige Körper (1) zylindrisch ist.

14. Knochenschraube gemäß einem der Ansprüche 1 bis 13 zusammen mit einem Instrument zum Einbringen der Knochenschraube, wobei das Instrument eine Eingreifstruktur (102, 103) umfasst, welche in die Eingreifstruktur (31, 310) der Knochenschraube eingreift und welche zum Führen eines Führungsdrahts ausgebildet ist.

15. Kombination gemäß Anspruch 14, wobei das Instrument ein Führungsloch (104) zum Hindurchführen des Führungsdrahts umfasst.

## Revendications

1. Vis à os comportant
un corps tubulaire (1) avec un filetage osseux (5) dans au moins une partie de sa paroi externe, le corps tubulaire ayant une première extrémité ouverte (2) et une deuxième extrémité ouverte (3) ;
dans laquelle au niveau de la première extrémité ouverte (2) une pluralité de dents de coupe (4) est prévue et
dans laquelle au niveau de la deuxième extrémité (3) une structure (31, 310) pour une mise en prise avec un outil de vissage (100) est prévue ; **caractérisée en ce que** les dents de coupe (4) sont plus épaisses dans une direction radiale par rapport à la paroi du corps tubulaire.

2. Vis à os selon la revendication 1, dans laquelle les dents de coupe (4) s'étendent de manière coaxiale à l'axe central (L) du corps tubulaire.

3. Vis à os selon la revendication 1 ou 2, dans laquelle les dents de coupe (4) sont configurées pour découper un trou lorsque le corps tubulaire (1) est vissé dans l'os.

4. Vis à os selon l'une des revendications 1 à 3, dans laquelle les dents de coupe (4) ont une forme substantiellement en dents de scie.

5. Vis à os selon l'une des revendications 1 à 4, dans laquelle les dents de coupe (4) forment un bord de la première extrémité ouverte (2).

6. Vis à os selon l'une des revendications 1 à 5, dans laquelle l'épaisseur de paroi du corps tubulaire est inférieure à environ 15% du diamètre externe du corps tubulaire.

7. Vis à os selon l'une des revendications 1 à 6, dans laquelle la structure de mise en prise comprend une pluralité d'évidements (31) s'étendant à partir de la deuxième extrémité ouverte (3) à travers la paroi du corps tubulaire de manière substantiellement coaxiale à un axe longitudinal central (L) du corps tubulaire.

8. Vis à os selon l'une des revendications 1 à 7, dans laquelle la structure de mise en prise comprend un évidement polygonal ou est un évidement en forme d'étoile (31) s'étendant à partir de la deuxième extrémité ouverte (3) dans la paroi interne du corps tubulaire.

9. Vis à os selon l'une des revendications 1 à 6, dans laquelle une section (300) du corps tubulaire (1) qui est non filetée est formée de manière adjacente à la deuxième extrémité ouverte (3), dans laquelle la section (300) comprend une pluralité de fentes (310) s'étendant à partir du bord de l'extrémité ouverte (3) vers une certaine distance dans la direction longitudinale du corps tubulaire (1), dans laquelle les fentes (310) s'étendent complètement à travers la paroi, et dans laquelle les fentes (310) forment une structure de mise en prise pour un outil.

10. Vis à os selon l'une des revendications 1 à 9, dans laquelle la paroi du corps tubulaire comprend une pluralité d'ouvertures (6).

11. Vis à os selon l'une des revendications 1 à 8 ou 10, dans laquelle le corps tubulaire comprend une section non filetée.

12. Vis à os selon l'une des revendications 1 à 11, dans laquelle un fil de guidage (8) qui peut être inséré dans le corps tubulaire pour guider l'insertion de la vis à os, est prévu.

13. Vis à os selon l'une des revendications 1 à 12, dans laquelle le corps tubulaire (1) est cylindrique.

14. Vis à os selon l'une des revendications 1 à 13 conjointement avec un outil pour insérer ladite vis à os, dans laquelle l'outil comprend une structure de mise en prise (102, 103) qui se met en prise avec la structure de mise en prise (31, 310) de la vis à os et qui est configurée pour guider un fil de guidage.

15. Combinaison selon la revendication 14, dans laquelle l'outil comprend un trou de guidage (104) pour guider le fil de guidage à travers celui-ci.
